Europäisches Patentamt

European Patent Office

Office européen des brevets

(19)

(11) Publication number: **0 173 648**
**A2**

## EUROPEAN PATENT APPLICATION

(21) Application number: 85810385.6

(22) Date of filing: 26.08.85

(51) Int. Cl.⁴: **C 12 P 21/00,** C 07 K 15/00,
C 12 N 15/00, C 12 N 5/00,
G 01 N 33/577, G 01 N 33/574,
A 61 K 39/395, A 61 K 49/02,
C 07 K 3/18, C 07 K 15/14
// (C12P21/00, C12R1:91)

(30) Priority: 30.08.84 GB 8421944
19.06.85 GB 8515538

(43) Date of publication of application: 05.03.86
Bulletin 86/10

(84) Designated Contracting States: **AT BE CH DE FR GB IT
LI LU NL SE**

(71) Applicant: **CIBA-GEIGY AG, Klybeckstrasse 141,
CH-4002 Basel (CH)**

(72) Inventor: **Rosenfelder, Günter, Dr., Am Sonnenrain 21,
D-7851 Binzen (DE)**
Inventor: **Towbin, Harry, Dr., Feldstrasse 31,
CH-4123 Allschwil (CH)**
Inventor: **Braun, Dietmar, Prof. Dr., Wyhlenweg 4,
CH-4126 Bettingen (CH)**

(54) **New monoclonal antibodies to glycoconjugates, processes for their production, and applications.**

(57) The invention concerns new monoclonal antibodies against cell membrane glycoconjugates, e.g. neutral glycosphingolipids produced by epithelial cells, derivatives thereof such as antibody fragments, radioactively labelled monoclonal antibodies, and conjugates of the monoclonal antibodies with cell toxins, radionuclide complexing agents, or enzymes, processes for the preparation of these antibodies and their derivatives, hybridoma cell lines that secrete these antibodies, a process for the preparation of such hybridoma, pharmaceutical preparations containing the monoclonal antibodies or their derivatives, and the use of the monoclonal antibodies and their derivatives in the diagnosis and treatment of cancer.

EP 0 173 648 A2

4-15051/1+2/=

New monoclonal antibodies to glycoconjugates, processes for their production, and applications.

The invention concerns new monoclonal antibodies against cell membrane glycoconjugates, derivatives thereof, processes for the preparation of these antibodies and their derivatives, hybridoma cell lines that secrete these antibodies, a process for the preparation of such hybridoma, pharmaceutical preparations containing the monoclonal antibodies or their derivatives, and the use of the monoclonal antibodies and their derivatives in the diagnosis and treatment of cancer.

Background of the invention

Glycoconjugates are bio-molecules containing a carbohydrate function, e.g. glycolipids such as glycosphingolipids, glycoproteins, either N- or O-glycosylated, and proteoglycanes. Glycosphingolipids (GSL) and glycoproteins are responsible for some of the manifold functions of biological membranes.

Glycosphingolipids are composed of three basic structural units: a base, a fatty acid, and a carbohydrate. The lipid moiety of GSL contains a long chain amino-alcohol, the most common being sphingosine, to which a fatty acid is linked via an amide bond. This structure is called ceramide. The hydrophilic carbohydrate unit is linked to the primary hydroxyl group of sphingosine by a glycosidic bond. The carbohydrate is a mono- or usually an oligosaccharide composed of D-glucose, D-galactose, D-mannose, L-fucose, N-acetyl-D-glucosamine, N-acetyl-D-galactosamine, and/or N-acetylneuraminic (sialic) acid. Such carbohydrate residues are often found also as components of membrane glycoproteins.

Glycosphingolipids are usually located in the outer leaflet of the plasma membrane. Several functions have been ascribed to them: They confer structural rigidity to membranes, are involved in ion transport through membranes, display receptor functions towards glycoprotein hormones, lymphokines, bacterial toxins and the like, and are cell surface antigens and markers involved in cell growth and cell interaction. The latter property has been studied in connection with tumourigenesis and metastasis. Alterations of GSL composition are associated with malignancy, and a few unique GSL antigens are found only in tumours.

Antibodies have been raised against tumour cell surface structures including these antigenic glycosphingolipids with the prospect of gaining valuable tools in tumour diagnosis and immunotherapeutics. Hakomori (Bulletin du Cancer, Paris, 70, 118 (1983)) reviews the glycolipid changes associated with oncogenic transformations and the use of monoclonal antibodies in this context.

German OLS 32 28 233 e.g. describes the use of an antibody R-24 for the treatment of tumours in human. This antibody has been raised against malignant melanoma cells and is specific for an antigen identified as a ganglioside, i.e. a sialic acid containing glycosphingolipid. Some of the antibodies described in U.S. Patent 4'444'744, which are raised against carcino-embryonic antigen, or in European Patent application 37 953, which are directed against malignant renal cells, may also be specific to glycosphingolipids shown to be integral components of the cell surface membrane. Due to the relationship of the carbohydrate residue of glycosphingolipids and membrane glycoproteins, antibodies raised against glycolipids may recognize also glycoproteins and vice versa.

The hybridoma technique of Köhler und Milstein (Nature 256, 495 (1975)) allows one to prepare homogeneous populations of highly specific antibodies, so-called monoclonal antibodies, reproducibly and in theoretically unlimited amounts from cell cultures. The fusion of myeloma cells with antibody-secreting lymphocytes taken from the spleen of a mammal previously immunized with a particular antigen yields hybrid cells, which combine the ability of unlimited replication and growth in vitro with the antibody-producing characteristics of the spleen cell. Therefore it is possible to isolate and propagate the immune answer of an individual organism to a specified antigen, and to produce monoclonal antibodies by permanent culture of hybrid cells in vitro.

While the general technique is well understood conceptually, there are many difficulties met and variations required for each specific case. In fact, there is no assurance that the desired hybridoma will be obtained, that they are genetically stable, secrete antibodies, or that the antibodies so produced will have the desired specificity. The degree of success is influenced principally by the type and purity of the antigen employed in the immunization, the method of immunization, the technique of cell fusion, and, above all, the method of selection of particular hybridoma cell clones.

Description of the invention

The invention concerns new monoclonal antibodies and derivatives thereof, characterized in that they bind to neutral glycosphingolipids produced by epithelial cells.

In particular the invention concerns monoclonal antibodies and derivatives thereof, characterized in that they bind to neutral glycosphingolipids produced by malignant epithelial cells, such as mammary, lung or colorectal carcinoma cells, especially mammary carcinoma cells. Preferred are also monoclonal antibodies and derivatives thereof, which bind to neutral glycosphingolipids obtained from meconium.

Most preferred are the monoclonal antibodies with the designation 1018S19.11, 1018S69.4, 1021S11.28, 1022S25.17 and 1022S23.24, and derivatives thereof, but particularly the monoclonal antibodies themselves.

Derivatives of monoclonal antibodies of this invention are e.g.
antibody fragments, radioactively labelled monoclonal antibodies,
and conjugates of the monoclonal antibodies with cell toxins,
radionuclide complexing agents, or enzymes.

Fragments of monoclonal antibodies of this invention are e.g. Fab,
Fab' or F(ab')$_2$ fragments, which retain their specificity for the
antigenic determinants, i.e. which retain the characteristic binding
pattern of the parent monoclonal antibody to glycosphingolipid
mixtures.

Radioactively labelled monoclonal antibodies contain e.g. radio-
active iodine ($^{123}$I, $^{125}$I, $^{131}$I), carbon ($^{14}$C), sulfur ($^{35}$S),
tritium ($^3$H) or the like. Preferred are monoclonal antibodies
labelled with radioactive iodine.

Antibody conjugates of the invention are e.g. conjugates of monoclonal antibodies or fragments thereof with cytotoxic and
carcinostatic agents such as the A chain of ricin or related
peptides, adriamycin, methotrexate, vincristin, daunomycin,
6-mercaptopurine, cytosine, arabinoside, cyclophosphamide, and the
like, or conjugates with metallocenes or metal chelates such as
dextran-amine, polyalkyleneimine carrying carboxylic or phosphonic
acid residues, e.g. diethylenetriaminepentaacetic acid, and the
like, in which the metal chelate complexes a radionuclide, e.g.
$^{113}$In, $^{111}$In, $^{207}$Bi, $^{46}$Sc, $^{195}$Pt, $^{57}$Ni, $^{57}$Co, $^{97}$Ru, $^{99}$Ru or $^{99}$Tc, or
conjugates with enzymes such as horseradish peroxidase, alkaline
phosphatase, ß-D-galactosidase, glucose-oxidase, glucoamylase,
carboanhydrase, acetylcholinesterase, lysozym, malate dehydrogenase
or glucose-6-phosphate dehydrogenase. In such conjugates the
antibody is bound to the cell toxin, the radionuclide complexing
agent or the enzymes directly or by the way of a spacer or linker
group. Preferred are conjugates of monoclonal antibodies with

cytotoxic agents connected <u>via</u> a bond or <u>via</u> a linker susceptible to
cleavage by one of the serum complement enzymes as described in
European Patent Application 88 695.

The monoclonal antibodies and their derivatives of this invention
bind to neutral glycosphingolipids produced by epithelial cells.
Such glycosphingolipids may be incorporated in the cell membrane of
the epithelial cells or may be released by them into the body
fluids.

The monoclonal antibodies of the invention are analyzed with regard
to their binding pattern towards glycosphingolipid mixtures of
varying composition, their binding ability towards tissue sections
and the cell types contained therein of normal and malignant tissue
samples from various organs, their binding ability towards whole
cells derived from established tumour cell lines, their binding
ability towards saliva from different donors, and their immunoglobulin class or subclass.

The binding pattern of monoclonal antibodies to glycosphingolipid
(GSL) mixtures can be determined e.g. by the so-called GSL-blotting
technique. The glycosphingolipid mixtures are obtained by known
methods, e.g. by solvent extraction, solvent distribution and
chromatographic methods, from various tissues, such as breast,
liver, kidney, spleen, tonsil tissue, also tumour tissues, e.g.
mammacarcinoma, lung tumours and the like, from erythrocytes and
other cellular sources, for instance meconium, i.e. the faeces of
newborns. The GSL mixture is separated by thin layer chromatography,
and the entire chromatographic pattern transferred to nitrocellulose. To that end, the thin layer plate is moistened with a suitable
solvent such as an aqueous lower alcohol and pressed on a sheet of
porous nitrocellulose. The obtained replica on nitrocellulose is
optionally cut into strips and incubated with solutions containing
monoclonal antibodies, and any immunological reaction of the
antibodies with the glycosphingolipids adsorbed on the nitrocellulose detected by methods known in the art, e.g. by development with a second antibody conjugated to an enzyme followed by an enzyme substrate or development with a radioactively labelled second antibody.

The monoclonal antibodies of this invention are analyzed by GSL-blotting with regard to their binding pattern towards GSL mixtures from breast tumour biopsy material derived from 19 individual humans. The monoclonal antibodies of the invention bind to neutral GSL in most of the GSL mixture tested, but do not bind to gangliosides, i.e. sialic acid containing GSL. The approximate number of monosaccharide units of the carbohydrate chain of the antigenic neutral GSL is shown in the Table.

The binding ability of the monoclonal antibodies of the invention towards different tissue sections and the cell types contained therein is determined by known methods, e.g. by immunohistochemical staining. To that end, cryosections of normal breast, liver, kidney, spleen, lung, colon and tonsil tissue, and of colorectal, mammary, lung and epithelial cyst tumour tissue are incubated with monoclonal antibodies of the invention and developed e.g. with an enzyme-conjugated second antibody, which recognizes and binds the monoclonal antibody of the invention. A suitable colour reaction of an enzyme substrate then makes visible the immune reaction of the monoclonal antibodies with the glycoconjugates contained in the tissue cell surface membrane. Without exception, binding is limited to epithelial cell membranes, i.e. not observed with membranes of other cell types such as fibroblasts, endothelium, erythrocytes, lymphocytes, muscle cells, and the like. The results are collected in the Table.

The binding ability of the monoclonal antibodies of the invention towards whole cells of established tumour cell lines of mammary, colorectal and lung carcinoma origin is determined likewise, i.e. by immunohistochemical staining.

The binding ability of the monoclonal antibodies of the invention towards saliva of different donors with known A,B,O and Lewis blood groups helps to reveal any putative relation between antigenic glycoconjugates found on malignant epithelial cells and known blood group markers expressed on saliva, and further is a simple means to show non-identity of the antibodies tested. The assay is based on a known dot-immunobinding assay with saliva dots on nitrocellulose sheets using a second enzyme-conjugated antibody, which recognizes and binds the monoclonal antibody of the invention.

The immunoglobulin class and subclass of the monoclonal antibodies of the invention is determined by well-known methods, e.g. the immuno-diffusion Ouchterlony technique using class-specific second antibodies.

Table: Characterization of monoclonal antibodies

| Antibody designation | 1018S 19.11 | 1018S 69.4 | 1021S 11.28 | 1022S 25.17 | 1022S 23.24 |
|---|---|---|---|---|---|
| Source of antigen used for immunization (Ex. 1 and 2) | ---mammary carcinoma--- | | | ---meconium--- | |
| Antibody class (Ex. 5) | $IgG_1$ | $IgG_3$ | $IgG_{2a}$ | $IgG_{2b}$ | $IgG_{2b}$ |
| Binding to GSL from different mammary biopsies (Ex. 6) | 11/19 | 17/19 | 11/16 | n.t. | 15/16 |
| Approx. number of monosaccharides in antigenic neutral GSL | 5 | 6 | 5 | 5 | 5 |
| Binding to epithelial cells of normal tissue section (Ex. 8) | | | | | |
| breast | 3/5 | 4/5 | 3/5 | 5/5 | 5/5 |
| liver | 0/1 | 1/1 | n.t. | n.t | n.t. |
| kidney | 0/1 | 1/1 | n.t. | n.t. | n.t. |
| spleen | 0/1 | 0/1 | n.t. | n.t. | n.t. |
| lung | 1/1 | 1/1 | 1/2 | 1/2 | 2/2 |
| colon | 4/5 | 3/5 | 4/5 | 5/5 | 5/5 |
| tonsil | 1/1 | 1/1 | n.t. | n.t. | n.t. |
| Binding to epithelial cells of tumour tissue sections (Ex. 8) | | | | | |
| colorectal | 11/12 | 1/3 | 2/5 | 5/5 | 4/4 |
| mammary | 4/6 | 2/4 | 1/5 | 2/4 | 4/4 |
| lung | 4/11 | 1/1 | n.t. | n.t. | n.t. |
| epithelial cyst | 1/1 | 1/1 | n.t. | n.t. | n.t. |
| Binding to tumour cell lines (Ex. 9) | | | | | |
| mammary: BT-20 | - | - | + | - | - |
| MCF-7 | - | + | - | - | - |
| ZR-75-1 | (+) | (+) | + | + | (+) |
| MDA-MB 231 | - | - | + | (+) | (+) |
| colorectal: SW 1222 NU | ++ | ++ | - | (+) | ++ |
| SW 948 NU | ++ | - | (+) | ++ | ++ |
| lung: A 549 | - | - | - | - | - |
| MBA 9812 | (+) | - | - | + | + |

| Antibody designation | 1018S 19.11 | 1018S 69.4 | 1021S 11.28 | 1022S 25.17 | 1022S 23.24 |
|---|---|---|---|---|---|
| Binding to saliva (Ex. 10) A, B, O blood group of saliva donors Lewis group of saliva donors | A,O a,b | A b | - - | A,B,O a,b | A,B,O a,b |

11/19 = 11 positive out of a total of 19

n.t. = not tested

- = no reaction

(+) = doubtful

+ = weak

++ = well-defined.

The monoclonal antibodies of the invention and derivatives thereof are obtained by processes known per se, characterized in that hybridoma cells secreting said monoclonal antibodies

a) are cultivated in vitro and the monoclonal antibodies isolated from the culture supernatant, or

b) are propagated in vivo in a suitable mammal and the monoclonal antibodies recovered from body fluids of said mammal, and, if desired,

c) the obtained monoclonal antibodies are transformed into a derivative thereof.

Suitable culture media for the in vitro cultivation according to process a) are standard culture media such as Dulbecco's Modified Eagle Medium or RPMI 1640 Medium, optionally replenished by a mammal serum, e.g. fetal calf serum. The isolation of the monoclonal antibodies is accomplished by precipitating the protein contained in the culture supernatants by ammonium sulfate or the like, followed by purifying the immunoglobulins by standard chromatographic methods, such as gel filtration, ion exchange chromatography, chromatography on DEAE cellulose, or immunoaffinity chromatography.

Large amounts of the desired monoclonal antibodies can be obtained by the propagation of hybridoma cells according to process b). Cell clones are injected into syngeneic mammals, which causes antibody-producing tumours to grow. After one to three weeks the desired monoclonal antibodies are recovered from body fluids of said mammal. As an example hybridoma cells derived from Balb/c mice are intraperitoneally injected into Balb/c mice optionally pretreated with a hydrocarbon such as pristane, and after one to two weeks, ascites fluid of these mice is collected. The desired monoclonal antibodies are isolated from the body fluids by methods known per se, e.g. by precipitating the proteins with ammonium sulfate or the like, followed by purifying the immunoglobulins by standard chromatographic methods, such as gel filtration, ion exchange chromatography, chromatography on DEAE cellulose, or immunoaffinity chromatography.

Fragments of monoclonal antibodies, for example Fab, Fab' or $F(ab')_2$ fragments, which retain their specificity towards the antigenic determinants of the glycosphingolipids, can be obtained from the monoclonal antibodies prepared according to process a) or b) by methods known per se, e.g. by digestion with enzymes such as pepsin or papain and/or cleavage of disulfide bonds by chemical reduction.

Monoclonal antibodies labelled with radioactive iodine are prepared by iodination methods known in the art, e.g. by labelling monoclonal antibodies with radioactive sodium or potassium iodide and a chemical oxidant such as sodium hypochlorite, chloramine T or the like, or an enzymatic oxidant such as lactoperoxidase or glucose oxidase and glucose. Radioactively labelled monoclonal antibodies of the invention are also prepared by adding radioactively labelled nutrients to the culture media of the in vitro cultivation of step a). Such labelled nutrients contain e.g. radioactive carbon $(^{14}C)$, tritium $(^{3}H)$, sulfur $(^{35}S)$ or the like, and are for example L-$(^{14}C)$-leucine, L-$(^{3}H)$-leucine or L-$(^{35}S)$-methionine.

Conjugates of monoclonal antibodies of the invention are prepared by methods known in the art, e.g. by reacting a monoclonal antibody prepared according to process a) or b) or a fragment thereof prepared as described hereinbefore with the desired conjugation partner, i.e. the cytotoxic or carcinostatic compound, the complexing metal chelate, or the enzyme, in the presence of a coupling agent, e.g. glutaraldehyde, periodate, N,N'-o-phenylenedimaleimide, N-(m-maleimidobenzoyloxy)-succinimide, N-(3-[2'-pyridyldithio]-propionoxy)-succinimide, N-ethyl-N'-(3-dimethylaminopropyl)-carbodiimide or the like. To prepare the preferred conjugates of monoclonal antibodies with cytotoxic agents connected <u>via</u> a linker susceptible to cleavage by one of the serum complement enzymes, one of the methods described in the European Patent Application 88 695 is used.

The invention further concerns hybridoma cell lines, characterized in that they secrete monoclonal antibodies which bind to neutral glycosphingolipids produced by epithelial cells.

In particular the invention concerns cell lines which are hybrids of myeloma cells and B lymphocytes of a mammal immunized with glycosphingolipids. Preferentially these cell lines are hybrids of mouse or rat myeloma cells and B lymphocytes of a syngeneic mouse or rat immunized with glycosphingolipids.

Particularly preferred are the hybridoma cell lines with the designation 1018S19.11, 1022S25.17, 1021S11.28, and 1018S69.4, which have been deposited on August 16, 1984, and the hybridoma cell line 1022S23.24, deposited on May 31, 1985, at the "Collection Nationale de Cultures de Microorganismes", Institut Pasteur, Paris, under the number I-331, I-332, I-333, I-334, and I-453, respectively. These hybridoma cell lines are hybrids of the mouse myeloma cell line Sp2/O-Ag14 and of B lymphocytes of the spleen of Balb/c mice immunized with glycosphingolipids. They are stable cell lines, which secrete the monoclonal antibodies with the designation

- 12 -

0173648

1018S19.11, 1022S25.17, 1021S11.28, 1018S69.4, and 1022S23.24, respectively. The cell lines may be kept in deep-frozen cultures and reactivated by thawing and optional re-cloning.

The invention concerns also a process for the production of hybridoma cell lines secreting monoclonal antibodies which bind to neutral glycosphingolipids produced by epithelial cells, characterized in that a suitable mammal is immunized with a glycosphingolipid mixture or antigenic derivatives thereof, antibody-producing cells of this mammal are fused with myeloma cells, the hybrid cells obtained in the fusion are cloned, and cell clones secreting the desired antibodies are selected.

Suitable antigens for the immunization of mammals in the process of the invention are glycosphingolipid (GSL) mixtures and/or conjugates of these GSL mixtures with immunogenic carriers. GSL mixtures are obtained by known methods, e.g. by solvent extraction with solvent mixtures containing a lower alkanol, such as methanol, and a halogenated lower alkane, such as chloroform, optionally in the presence of water, from various tissues, such as breast, liver, kidney, spleen, tonsil tissues, also tumour tissue, e.g. mammary carcinoma, lung tumours and the like, from eryhtrocytes and other cellular sources, e.g. meconium, i.e. the faeces of newborns, optionally pretreated with an organic solvent such as acetone to remove storage fat. Preferred sources of GSL mixture are tissues of human origin, especially malignant tissues, such as mammary carcinoma biopsy material, or meconium. Optionally the crude GSL extracts are enriched and purified by solvent distrubtion between water optionally containing inorganic salts, such as sodium or potassium chloride, and organic solvent mixtures containing a lower alcohol such as methanol, and a halogenated lower alkane, such as chloroform, and/or by chromatographic methods, e.g. by column chromatography on normal or reversed phase silica gel, ion exchange resin, and the like.

Preferred are GSL mixtures obtained from mammary carcinoma pretreated with acetone or from meconium by extraction with methanol/chloroform/water-mixtures, solvent partition between water and methanol/chloroform, and chromatography over reversed phase silica gel.

Antigenic conjugates of GSL mixtures with immunogenic carriers are conjugates with e.g. high molecular weight polypeptides such as serum albumin, latex particles, whole cells or bacteria. They are prepared by methods known per se, either by adsorption of the GSL on the carrier or by coupling using carbodiimides, periodate, glutaraldehyde, N,N'-o-phenylenedimaleimide, N-(m-maleimidobenzoyloxy)-succinimide, N-(3-[2'-pyridyldithio]-propionoxy)-succinimide or the like. Preferred antigens are GSL mixtures adsorbed on bacteria, in particular on bacteria with a lipophilic surface, such as Salmonella minnesota R595 rough mutant bacteria. The ratio of dry weight Salmonella minnesota R595 to the weight of GSL mixtures is preferably between 2:1 and 10:1.

Preferred mammals for the immunization are mice or rats. Particularly preferred are Balb/c mice. The immunizations are performed e.g. by injecting GSL conjugates three to eight times parenterally, such as intraperitoneally and/or subcutaneously, at intervals of one to ten days, in amounts of about 100 µg to about 500 µg calculated on pure GSL mixture, followed by a booster injection of about 300 µg to about 600 µg GSL after two to eight weeks. The injections optionally contain an adjuvant stimulating the lymphocyte production such as complete or incomplete Freund's adjuvant, and such an adjuvant is indispensable if unconjugated GSL mixtures are injected in place of GSL conjugates. For injections of GSL mixtures adsorbed on bacteria, no additional adjuvant is given.

Antibody-producing cells of the immunized mammals, preferably spleen cells, taken two to five days after the final booster injection, are fused with myeloma cells of a suitable cell line in the presence of a fusion promoter. Several suitable myeloma cell lines are known in the art. Preferred are myeloma cell lines lacking the enzyme hypoxanthine guanine phosphoribosyl transferase (HGPRT) or the enzyme thymidine kinase (TK), which therefore do not survive in a selective culture medium containing hypoxanthine, aminopterin and thymidine (HAT medium). Particularly preferred are myeloma cells and derived cell lines that do not survive in HAT medium and do not secrete immunoglobulins or fragments thereof, such as the cell lines X63-Ag8.653 or Sp2/0-Ag14. Fusion promoters considered are e.g. Sendai virus or other paramyxo viruses, optionally in UV-inactivated form, calcium ions, surface-active lipids such as lysolecithin, or polyethylene glycol. Preferentially, the myeloma cells are fused with a three- to twentyfold excess of spleen cells from immunized mammals in a solution containing about 30 % to about 60 % polyethylene glycol of a molecular weight between 1000 and 4000.

After the fusion, the cells are resuspended and cultivated in selective HAT medium. Thereby, only hybridoma cells will survive, because they combine the ability to grow and replicate in vitro inherited from myeloma cells and the missing HGPRT or TK genes essential for the survival in the HAT medium inherited from the antibody-producing spleen cells of the immunized mammals.

Suitable culture media for the expansion of hybridoma cells are the standard culture media, such as Dulbecco's modified Eagle medium, minimum essential medium RPMI 1640 medium and the like, optionally replenished by serum, e.g. 10 to 15 % fetal calf serum. Preferentially feeder cells are added at the beginning of the cell growth, e.g. normal mouse peritoneal exsudate cells, spleen cells, marrow bone macrophages, or the like. The culture media are supplemented with selective HAT medium at regular intervals in order to prevent normal myeloma cells overgrowing the hybridoma cells.

The hybridoma cell culture supernatants are screened for the desired monoclonal antibodies, preferentially with an enzyme immunoassay or a radioimmunoassay, e.g. by GSL blotting. Positive hybridoma cells are cloned, e.g. by limiting dilution, preferentially twice or more. The cloned cell lines may be frozen in a conventional manner.

The monoclonal antibodies of the invention and/or their derivatives are useful for the qualitative and quantitative determination and/or purification of glycoconjugates from epithelial cell membranes.

For instance, the monoclonal antibodies or derivatives thereof, such as enzyme conjugates or radioactive derivatives, can be used in any of the known immunoassays, which rely on the binding interaction between the antigenic determinant, e.g. of a glycosphingolipid, and the monoclonal antibodies. Examples of such assays are radioimmunoassays, enzyme immunoassays, immuno' fluorescence, latex agglutination, and hemagglutination. Such immunoassays are useful e.g. in the diagnosis of oncogenic transformations resulting in a qualitative and quantitative change of glycoconjugate content of epithelial cell membranes.

Glycoconjugates, such as glycoproteins, from epithelial cell membranes can be purified by immunoaffinity chromatography with the aid of monoclonal antibodies of the invention. The monoclonal antibodies or fragments thereof are bound to a suitable carrier of organic or inorganic origin, such as cross-linked agarose, dextran or polyacrylamide in suitably functionalized form, optionally after activation, applying general methods known in the art. For instance, a carrier bearing activated ester functions is suspended in an aqueous buffer solution, mixed with a solution of a monoclonal antibody or a fragment thereof, filtered, resuspended and washed to remove excess monoclonal antibody, and treated with a solution of irrelevant proteins to block free reactive positions of the carrier. This antibody-coated carrier is used to selectively bind glycoconjugates from epithelial cell membranes. Desorption with various buffer solutions optionally containing additional inorganic salts then gives fractions containing the glycoconjugates of interest in purified form.

The monoclonal antibodies of the invention and/or their derivatives are further useful in the diagnosis and treatment of cancer.

For instance, radiolabelled derivatives of·the monoclonal antibodies of the invention can be used to detect primary and metastatic tumours in mammals by radioscanning techniques. To that end, the radioactive antibody is injected e.g. intravenously and the mammal scanned with a gamma imager at regular intervals. Tumours expressing antigenic GSL will take up more radioactive antibodies than other tissue and will be clearly recognized by the gamma imaging camera. Preferentially monoclonal antibodies labelled with $^{131}$I are used for radioscanning in amounts of 3 to 8 µg representing 15 to 30 µCi per kg body weight.

Further, the monoclonal antibodies themselves and particularly derivatives thereof such as conjugates with cytotoxic and carcinostatic compounds can be used for the treatment of cancer, especially mammary carcinoma which express the antigenic GSL. The therapeutic dose for mammals is between approximatively 1 mg and 10 mg per kg body weight for monoclonal antibodies themselves, and between 0.1 mg and 10 mg per kg body weight for conjugates with cytotoxic drugs, depending on the status of the patient and the mode of application.

The monoclonal antibodies and/or derivatives thereof are also useful in the immunological removal of cancer cells in bone marrow auto-grafts. To that end bone marrow aspirates of patients receiving high doses of drugs and/or radiation treatment are incubated with the monoclonal antibodies of the invention in the presence of complement, incubated with conjugates of monoclonal antibodies with cytotoxic drugs, or treated with monoclonal antibodies conjugated to magnetic microspheres as described by J.G. Treleaven et al. (Lancet, 1984, 70). This treatment will lyse or remove tumour cells expressing antigenic GSL without afflicting bone marrow stem cells, which then can be used for autologous transplantation.

The invention concerns also pharmaceutical preparations containing monoclonal antibodies binding to neutral glycosphingolipids produced by epithelial cells, or derivatives thereof, in a therapeutically effective amount together with a pharmaceutical carrier, solid or liquid, of organic or inorganic origin.

Preferred are pharmaceutical preparations for parenteral application. Preparations for intramuscular, subcutaneous or intravenous application are e.g. isotonic aqueous solutions or suspensions, optionally prepared shortly before use from lyophilized or concentrated preparations. The pharmaceutical preparations may be sterilized and contain adjuvants e.g. for conserving, stabilizing, wetting, emulsifying or solubilizing the ingredients, salts for the regulation of the osmotic pressure, buffer and/or compounds regulating the viscosity, e.g. sodium carboxycellulose, dextran, polyvinylpyrrolidone or gelatine. They are prepared by methods known in the art, e.g. by conventional mixing, dissolving or lyophilizing, and contain from approximately 0.01 % to approximately 50 % of active ingredients. The preparations for injections are processed, filled into ampoules or vials, and sealed under aseptic conditions according to methods known in the art.

The following examples illustrate the invention, but do not limit it to any extent.

The abbreviations used in the examples have the following meaning:

AEC     3-amino-9-ethylcarbazol

GSL     glycosphingolipids

HAT     hypoxanthine/aminopterin/thymidine

HPTLC     high performance thin layer chromatography

PBS     phosphate buffered saline

TBS     tris-(hydroxymethyl)-aminomethane buffered saline

Tris     tris-(hydroxymethyl)-aminomethane


Example 1: Isolation and purification of polar glycosphingolipids
from mammary carcinoma biopsy material.


1.1 Extraction

Cellular sediments derived from biospy tissue specimen taken for estrogen receptor determinations are obtained from a large number of patients with histologically confirmed diagnosis of mammary carcinoma.

In a typical experiment, 190 individual samples obtained as frozen clots in polystyrene tubes are further cooled down in dry ice, and the plastic is smashed with a hammer. The collected sediments (169 g wet weight) are lyophilized and yield 33 g dry weight. The dry material is freed from storage fat by repeated homogenization in acetone at 4°C using a Sorval tissue blender and subsequent filtration. The residual solid material still contains the bulk of polar lipids such as phospholipids and glycosphingolipids. These are extracted at room temperature three times using 200 ml of chloroform:methanol 2:1 (v/v) and twice with 200 ml chloroform:methanol:water 1:1:0.2 (v/v/v).

1.2 Folch partition

The bulk of phospholipids and those glycosphingolipids having less than four sugars in their oligosaccharide chain are separated from polar glycosphingolipids by a modified partition procedure according to Folch (T. Saito and S. Hakomori, J. Lipid Res. 12, 257 (1971)). The collected extracts from example 1.1 are evaporated under reduced pressure at 40°C and redissolved in 90 ml of chloroform:methanol 2:1 (v/v) in a stoppered glass cylinder. 15 ml of water are added and the solutions are vigorously shaken. After 15 min standing the phases have separated. The lower chloroform-rich phase is extracted three times with chloroform:methanol: 0,1 % aqueous KCl 1:10:10 (v/v/v).

## 1.3 Reversed phase chromatography

To remove salt and other water-soluble contaminants, the combined upper phases from example 1.2 are evaporated, taken up in water and applied to a glass column containing 7 ml of Lichroprep® RP18 (Merck, F.R.G.). After extensive washing with water the polar glycosphingolipid fraction is eluted with 10 ml of methanol and then 10 ml of chloroform:methanol 2:1 (v/v). The yield is about 30 mg, i.e. 0.1 % of tissue dry weight.

## 1.4 Thin layer chromatography

The purity of the GSL preparation is tested by silica gel thin layer chromatography and subsequent spraying of the plates with either orcinol/sulphuric acid reagent visualizing sugar containing compounds (L. Svennerholm, J. Neurochem. 1, 42 (1956)) or Phospray® (Supelco Inc., Bellefonte, Pa., USA, see J.C. Dittmer and R.L. Lester, J. Lipid Res. 5, 126 (1964)) to detect phospholipids. Silica gel 60 HPTLC plates (size 5x5 cm, thickness of sorbent layer 0.24 mm, Merck, F.R.G.) are used. The lipid samples are dissolved in 10-20 μl of chloroform:methanol 2:1 (v/v) and applied to the plates as a streak of 4 cm length using an automated sample applicator model Linomat III (Camag, Switzerland). The solvent system is chloroform:methanol:water 55:45:10 (v/v/v) containing 1 mM $CaCl_2$. The developed plates show the typical pattern of GSL mixtures with. carbohydrate residues of varying monosaccharide content. Only traces of non-glycosphingolipid material are present.

Example 2: Isolation and purification of glycosphingolipids from
         meconium

Newborn faeces from various individuals are scraped from diapers and
treated exactly the same way as described in example 1 with the
exception that the acetone extraction step of example 1.1 is
omitted. The yield is about 0.2 % of dry weight. The composition and
purity of the glycosphingolipid (GSL) mixture is determined as
described in example 1.4.


Example 3: Production of hybridoma cells


3.1 Preparation of the immunogen

Purified GSL from example 1 or 2 are adsorbed to freeze dried and
acetic acid-treated Salmonella minnesota R-595 bacteria (C. Galanos,
O. Lüderitz and O. Westphal, Eur. J. Biochem. 24, 116 (1971)) to
increase the immunogenicity of the lipid antigens. To that end,
50 mg of bacteria (dry weight) are suspended in a 250 ml round
bottom flask in 100 ml of chloroform:methanol 2:1 (v/v) containing
10 mg of GSL. The solvent is evaporated at room temperature using a
rotary evaporator revolving at full speed. GSL covered bacteria are
scraped from the glass wall and suspended in phosphate buffered
saline (PBS) at a concentration of 2.4 mg/ml. Upon microscopial
evaluation irregularly shaped clumps as well as single bacteria are
observed.


3.2 Immunization protocol

4 female Balb/c mice are injected intraperitoneally each with
samples of GSL-covered bacteria containing 100 µg of GSL on day 0,
200 µg on day 4, 300 µg on day 7, and 400 µg on day 11. On day 19
the mice are bled orbitally and tested for anti-GSL serum activity
using the GSL-blotting system described in example 6. On day 36 a
booster injection of a sample containing 400 µg GSL is given, and
the mice are sacrificed four days latter.

3.3 Cell fusion

All fusion experiments are performed according to the procedure of
G. Köhler and C. Milstein (Nature 256, 495 (1975)) using the
nonsecreting Sp 2/0-Ag14 myeloma line (M. Shulman, C.D. Wilde and
G. Köhler, Nature 276, 269 (1978)). $10^8$ spleen cells are mixed with
$10^7$ myeloma cells in the presence of 1 ml of 50 % polyethylene
glycol (PEG 1500, Serva). After washing, the cells are resuspended
in 48 ml of standard Dulbecco's minimum essential medium (Gibco
No. 0422501). 15 % fetal calf serum and $3x10^6$ normal mouse peri-
toneal exsudate cells per fusion are added as feeder cells. The
cells are distributed into 48 x 1 ml costar wells and fed 3 to 4
times per week with standard HAT selection medium for 3 to 6 weeks.
When the growth of hybridoma cells becomes visible, the supernatants
are tested for binding to GSL (example 6). Cloning of hybridoma
cells is done by limiting dilution in microtiter plates. The
supernates are tested for binding to GSL in a solid phase enzyme
immunoassay described in example 7. All hybridoma lines are cloned
at least once.

Example 4: Isolation and purification of monoclonal antibodies Balb/c mice 8-10 weeks of age (Tierfarm Sisseln, Switzerland) are
pretreated intraperitoneally with 0.3 ml pristane (Aldrich). 2-3
weeks later, $2-5x10^6$ cloned hybridoma cells and 0.2 ml pristane are
inoculated intraperitoneally. After 8-10 days ascites fluid is
collected, centrifuged at 800 x g and stored at -20°C.

Defrosted ascites fluid is centrifuged at 50000 x g for 60 min. A
fat layer floating on the surface is carefully removed, and the
protein concentration is adjusted to a concentration of 10-12 mg/ml.
Crude immunoglobulin is precipitated by dropwise addition of
0.9 volume equivalents of saturated ammonium sulphate at 0°C, then
dissolved in 20 mM Tris-HCl/50 mM NaCl (pH 7.9) and dialysed against
the same buffer. An immunoglobulin G fraction is obtained by
DEAE-D52 cellulose (Whatman) chromatography using a buffer gradient

0173648

system of 20 mM Tris-HCl/25-400 mM NaCl, pH 7.9. The immunoglobulin G is again precipitated with ammonium sulphate and dissolved in
PBS at a concentration of 10 mg/ml.

Sodium dodecyl sulphate polyacrylamide gel electrophoresis
(SDS-PAGE) demonstrates a purity grade of more than 95 percent for
the monoclonal antibodies 1018S19.11, 1018S69.4, 1021S11.28,
1022S25.17, and 1022S23.24.

Example 5: Determination of the class and subclass of monoclonal
antibodies.

The class and subclass of monoclonal antibodies produced by cloned
hybridoma cells is determined in an enzyme immunoassay. Microtiter
plates are coated with 1 µg per well of a rabbit immunoglobulin
preparation of a class- or subclass-specific serum in 50 µl of PBS.
Free binding capacity of the plate is saturated with a buffer of 1 %
bovine serum albumin in PBS containing 0.2 % NaN₃ (w/v), pH 7.4.
100 µl samples containing monoclonal antibodies are incubated in the
wells at 37°C for 1 h. The plates are washed with PBS, then incubated at 37°C for 1 h with a phosphatase conjugated rabbit immunoglobulin preparation of the specificity as used for coating the plates.
The fixed enzyme is developed by incubating (37°C, 30 min) with a
solution of the enzyme substrate p-nitrophenyl phosphate (1 mg/ml in
diethanolamine buffer 10 % containing 0.5 mM MgCl₂ and 0.02 % (w/v)
NaN₃, pH 9.8) and measuring the optical density at 405 nm.

Example 6: Characterization of the specificity of monoclonal
antibodies against GSL by GSL-blotting

The GSL mixture is separated by thin layer chromatography on HPTLC
plates as described in example 1.4, then a replica is prepared from
the plate by transferring the entire pattern of separated GSL to
nitrocellulose. This GSL-blotting assay is especially convenient for
screening purposes. Specifically, 100 µg of human breast biopsy GSL
prepared according to example 1 are applied as a 10 cm long streak
to a HPTLC plate size 5x10 cm. After separation in the solvent
system of example 1.4, the plate is dried. Using a soft pencil,

lines are drawn as markers for later orientation. The plate is sprayed with a solution of isopropanol/water (2:1) until it is just visibly wet without formation of droplets on the surface. The plate is immediately pressed for 1 min on a slightly larger nitrocellulose sheet (HAWP00®, Millipore) supported by a sheet of polyethylene. Excess nitrocellulose is cut off and the replica is allowed to dry. The sheet is rehydrated in a buffer containing 20 mM Tris-HCl/150 mM NaCl/5 mM $MgCl_2$/0.15 mM $CaCl_2$, pH 7.4 (TBS). The sheet is then incubated with 10 % horse serum in TBS for 1 h at 40°C to block excess binding capacity.

The sheet is cut into strips of 2 mm width, each carrying the same pattern of separated GSL. The strips are incubated for 2 h e.g. with hybridoma supernatants from example 3.3 in multipipet reservoir inserts (Dynatech Laboratories Inc., Alexandria, Va., USA) serving as suitable incubation trays. The strips are washed three times for 5 min with TBS and incubated for a further 1 h with a conjugate of horseradish peroxidase and sheep immunoglobulin directed against mouse IgG (Cappel Laboratories Inc., Chochranville, USA) diluted 500-fold with 10 % (v/v) horse serum in TBS. After washing in TBS as above, the bound conjugate is visualized by the peroxidase catalyzed oxidation of 4-chloro-1-naphthol to a blue insoluble deposit. The substrate 4-chloro-1-naphthol is diluted 50-fold in TBS from a 1 % (w/v) solution in dimethyl formamide, and hydrogen peroxide is added to a final concentration of 0.006 % (w/v). After 20 min the reaction is stopped by washing with water. In the case of a positive reaction one or several bands are seen. Different staining patterns suggest the occurence of different antibodies.

For the GSL-blotting assay used in the characterization of indivi- dual antibodies, 5 µg of human breast biopsy GSL from a tissue specimen of single patients is applied to a HPTLC plate and processed as described above.

Example 7: <u>Screening for antibodies against GSL by solid phase</u>
<u>enzyme linked immunosorbent assay (ELISA)</u>

A 96-well polyvinylchloride microtiter plate is coated with 200 ng
per well of a GSL extract from breast carcinoma biopsy material
prepared as in example 1 dissolved in 25 µl ethanol. The wells are
allowed to dry and are completely filled with 1 % (v/v) horse serum
in TBS (composition of TBS cf. example 6). After 1 h the plate is
washed with TBS, and 50 µl of a solution to be tested for monoclonal
antibodies is added. After 3 h the plate is washed and 50 µl of a
reagent containing an enzyme-linked second antibody are added. This
reagent consists of alkaline phopshatase conjugated goat antibodies
against mouse immunoglobulins in a 1000-fold dilution in 1 % (v/v)
horse serum in TBS. After 1 h the plate is washed 4 times with TBS
and developed with the enzyme substrate p-nitrophenyl phosphate in
10 % diethanolamine buffer as described in example 5.


Example 8: <u>Immunohistochemical staining with monoclonal antibodies</u>
<u>against GSL</u>

Cryosections of 5 to 10 µm are prepared in the usual way from
cryopreserved or formalin-fixed normal breast, liver, kidney,
spleen, lung, colon and tonsil tissue and tissue from colorectal,
mammary and lung carcinoma or epithelial cyst tumours. Paraffin
embedded tissue is not suitable due to the danger of loosing GSL
during the incubations in solvents containing alcohol. The
cryosections are dried at 50°C for 5 min, defatted in ice-cold
acetone for 5 min, dried again and preincubated with 10 % (v/v)
horse serum in PBS. After 30 min the slides are washed with PBS for
5 min. Hybridoma supernatant or another sample to be tested for
monoclonal antibodies (about 400 µl, sufficient to cover the tissue
section) is added. After 1 h the slide is washed 3 times with PBS,
and the peroxidase conjugated second antibody described in example 6
is added. After 30 min the slides are washed as above and developed
with 3-amino-9-ethylcarbazol (AEC) yielding a red deposit. The
enzyme substrate solution consists of a freshly prepared and
filtered solution of 4 mg/ml AEC in dimethyl formamide diluted
20-fold with sodium acetate buffer prepared by mixing 100 parts 0.1 M aqueous sodium acetate and 40 parts 0.1 M aqueous acetic acid. Hydrogen peroxide concentration is adjusted to 0.014 % (w/v). The slides are incubated for 15 min in the dark, washed 3 times with acetate buffer and dried. The sections are mounted in glycerol-gelatine, pH 5.0, and inspected with a microscope. Suitable controls have to be included consisting of sections incubated with second antibody only, irrelevant first antibody, and no antibody at all to assess endogenous peroxidase activity.

Example 9: Cell surface staining on live cells

Adherent carcinoma cell lines of mammary, colorectal and lung origin are grown in 96 well tissue culture plates according to standard tissue culture technology. The cells are incubated with 100 µl each of hybridoma supernatants or other samples to be tested for monoclonal antibodies, then washed, incubated with a peroxidase conjugated second antibody, and developed with AEC as described in example 8. In order to avoid loosing cells, the washing steps have to be done carefully without removing the liquid completely. The stained cells are inspected with an inverted microscope immediately after the last washing step following development with AEC.

Details about the cell lines tested may be found in the following literature references: BT-20: E.Y. Lasfargues and L. Ozzello, J. Natl. Cancer Inst. 21, 1131 (1958); MCF-7: H.D. Soele et al., J. Natl. Cancer Inst. 51, 1409 (1973); ZR-75-1: R. Cailleau et al., J. Natl. Cancer Inst. 53, 661 (1974); MDA-MB 231: L.W. Engel et al., Cancer Res. 38, 3352 (1978); SW 1222 NU and SW 948 NU: A. Leibovitz in "Human tumor cells in vitro" (J. Fogh, ed.), Plenum Press, New York, 1975, p. 23; A 549: M. Lieber et al., Int. J. Cancer 17, 62 (1976); MBA 9812: G.J. Todaro, C. Fryling and J.E. DeLarco, Proc. Natl. Acad. Sci. U.S.A. 77, 5258 (1980).

Example 10: Saliva dot assay

The procedure is an adaption of the known dot-immunobinding assay
described in the European Patent Application EP 63 810 to saliva as
antigen. Individually collected saliva samples from donors of known
A,B,0 and Lewis blood groups are heated for 10 min at 100°C, and
centrifuged at 10'000 xg for 10 min. The supernatant (undiluted,
diluted 1:3 and 1:10 in PBS) is applied in 0.2 μl aliquots to
nitrocellulose (with a grid printed on it, Millipore, Bedford, MA).
The sheet is blocked with 10 % horse serum in PBS for 1 hour at
40°C, cut into strips in such a way as to contain the entire panel
of saliva samples, incubated with the monoclonal antibodies (ascitic
fluid from example 4 diluted 1:500 or culture supernatant from
example 3.3) for 3 hours, washed, and incubated with a peroxidase
coupled goat antibody raised against mouse immunoglobulin (Nordic
Laboratories, Tillburg, Netherlands, diluted 1:500) for 1 hour. The
peroxidase is detected with 4-chloro-1-naphthol and $H_2O_2$ as
described in example 6.

Example 11: Preparation of a conjugate of monoclonal antibody
1018S19.11 with ricin A

To a stirred solution of 3.0 mg monoclonal antibody 1018S19.11 in
1.5 ml of PBS at room temperature, a solution of 0.187 mg N-succinimidyl 3-(2-pyridyldithio)propionate (Pharmacia) is added. After
30 min, the mixture is dialysed against PBS at 4°C. The activated
antibody is mixed with 0.5 ml of a PBS solution containing 3.0 mg
ricin A purified by gel chromatography on Sephadex® G25, and kept at
room temperature for 20 hr. The reaction mixture is chromatographed
on a Sephadex® G 100 gel column. The fractions are analyzed for
antibody content by measuring the optical density at 280 nm, and for
ricin A by its inhibitory power of protein synthesis measured in an
acellular system, and identical fractions are combined to give about
1 mg of conjugate with an average of about 1.5 mol ricin A per mol
antibody in the conjugate.

Example 12: Preparation of $^{125}$I labelled antibody 1018S19.11

40 μg antibody 1018S19.11 are iodinated with 0.5 mCi $^{125}$I sodium iodide and chloramine T following the general procedure of F.C. Greenwood et al., Biochem. J. 89, 114 (1963). The reaction product is purified by chromatography on ion exchange resin Bio Rad AG 1 x 8®.

Example 13: Localization of human tumours in nude mice with radiolabelled monoclonal antibodies

13.1 Scintigraphy

Transplantable human breast carcinomas are grown subcutaneously in nude mice as described by H.H. Fiebig & G.W. Löhr, Medwelt 1/2, 92-106 (1984). Potassium iodide (0.05 %) is added to the drinking water of the mice. After two days, 30 μCi (ca. 4 μg) $^{125}$I-labelled antibody 1018S19.11 (example 12) is administered intravenously, and two to ten days later, scintigraphy is performed using a gamma-camera according to Anger. During scintigraphy the animal is anesthetized with Nembutal® (Abbott) at 0.04 mg/g body weight. The radiolabelled antibody distinctly accumulates on the scintigram in the region of the macroscopically visible tumour. This accumulation is compared with regions of the mouse known to be tumour free and with animals treated with an irrelevant radiolabelled antibody known not to bind to the tumour.

13.2 Radioactivity of excised organs

The degree of localization of the radiolabelled antibody is determined by direct counting of radioactivity of the tumour and comparison to the radioactivity of other tissues. The specific radioactivity (radioactivity per weight of tissue in cpm/mg) found in the organs and fluids of an animal treated as described in example 13.1 is 1050 (breast carcinoma tumour), 70 (spleen), 107 (kidney), 182 (lung), 58 (intestine), 106 (liver), and 488 (blood). These figures are compared also to the specific radioactivity found in organs and blood from animals treated with an irrelevant radiolabelled antibody.

13.3 Autoradiography of cryosections of excised tissues Cryosections of uniform thickness (10 to 40 µm) are prepared from the excised organs and tumours. The slides are dried at 50°C for 5 min, washed for 5 min in PBS, dried, and contacted with an X-ray film (Ultrofilm-$^3$H®, LKB, Bromma, Sweden). The degree of localization of the tumour is judged from the blackening of the developed film compared to film exposed to normal tissues and to tissues from animals treated with irrelevant radiolabelled antibody. Typically, uniform faint blackening is observed on film exposed to normal tissues, and intense, often patchy blackening is seen on film exposed to tumour tissues of animals treated as described in example 13.1.

Example 14: Injection solution

120 mg monoclonal antibody 1018S19.11 prepared according to example 4 are dissolved in 5 ml physiological saline. The solution is passed through a bacteriological filter, and the filtrate filled in an ampoule under aseptic conditions. The ampoule is preferentially stored in the cold, e.g. at -20°C.

Ampoules containing monoclonal antibodies 1018S69.4, 1021S11.28, 1022S25.17, 1022S23.24 and the conjugate of antibody 1018S19.11 with ricin A (example 11) are prepared likewise.

Claims for the Contracting States: DE, GB, FR, CH, LI, IT, NL, BE, SE, LU

1. Monoclonal antibodies and derivatives thereof, characterized in that they bind to neutral glycosphingolipids produced by epithelial cells.

2. Monoclonal antibodies and derivatives thereof as claimed in claim 1, characterized in that they bind to neutral glycosphingolipids produced by malignant epithelial cells.

3. Monoclonal antibodies and derivatives thereof as claimed in claim 1, characterized in that they bind to neutral glycosphingolipids produced by mammary carcinoma cells.

4. Monoclonal antibodies and derivatives thereof as claimed in claim 1, characterized in that they bind to neutral glycosphingolipids obtained from meconium.

5. The monoclonal antibodies with the designation 1018S19.11, 1018S69.4, 1021S11.28 and 1022S25.17, and derivatives thereof, as claimed in claim 1.

6. The monoclonal antibodies with the designation 1018S19.11, 1018S69.4, 1021S11.28 and 1022S25.17 as claimed in claim 1.

7. The monoclonal antibody with the designation 1022S23.24, and derivatives thereof, as claimed in claim 1.

8. The monoclonal antibody with the designation 1022S23.24 as claimed in claim 1.

9. A process for the preparation of monoclonal antibodies and derivatives thereof as claimed in claim 1, characterized in that hybridoma cells secreting said monoclonal antibodies

a) are cultivated in vitro and the monoclonal antibodies isolated from the culture supernatant, or

b) are propagated in vivo in a suitable mammal and the monoclonal antibodies recovered from body fluids of said mammal, and, if desired,

c) the obtained monoclonal antibodies are transformed into a derivative thereof.

10. A process as claimed in claim 8, characterized in that hybridoma cells derived from Balb/c mice secreting the desired antibodies are intraperitoneally injected into Balb/c mice optionally pretreated with a hydrocarbon, after one to two weeks, ascites fluid of these mice is collected, and the monoclonal antibodies are isolated therefrom by precipitating with ammonium sulfate and by standard chromatographic methods.

11. Hybridoma cell lines, characterized in that they secrete monoclonal antibodies as claimed in claim 1.

12. Hybridoma cell lines as claimed in claim 11, characterized in that they are hybrids of mouse or rat myeloma cells and B lymphocytes of a syngeneic mouse or rat immunized with glycosphingolipids.

13. Hybridoma cell lines as claimed in claim 11, characterized in that they are hybrids of the mouse myeloma cell line Sp2/O-Ag14 and of B lymphocytes of the spleen of Balb/c mice immunized with glycosphingolipids.

14. The hybridoma cell lines with the designation 1018S19.11, 1022S25.17, 1021S11.28 and 1018S69.4, which have been deposited at the "Collection Nationale de Cultures de Microorganismes", Institut Pasteur, Paris, under the number I-331, I-332, I-333, and I-334, respectively.

15. The hybridoma cell line with the designation 1022S23.24, which has been deposited at the "Collection Nationale de Cultures de Microorganismes", Institut Pasteur, Paris, under the number I-453.

16. A process for the preparation of hybridoma cell lines as claimed in claim 11, characterized in that a suitable mammal is immunized with a glycosphingolipid mixture or antigenic derivatives thereof, antibody-producing cells of this mammal are fused with myeloma cells, the hybrid cells obtained in the fusion are cloned, and cell clones secreting the desired antibodies are selected.

17. A process as claimed in claim 16, characterized in that Balb/c mice are immunized with glycosphingolipid mixtures adsorbed on Salmonella minnesota R 595 rough mutant by three to eight parenteral injections containing from 100 µg to 500 µg glycosphingolipid mixture at intervals of one to ten days, spleen cells of the immunized mice are taken two to five days after the final booster injection, and these cells are fused with myeloma cells of a suitable cell line in the presence of a fusion promoter.

18. A process as claimed in claim 16, characterized in that antibody-producing cells derived from immunized Balb/c mice are fused with myeloma cells of the cell lines X63-Ag8.653 or Sp2/0-Ag14.

19. A process as claimed in claim 16, characterized in that the myeloma cells are fused with a three- to twentyfold excess of spleen cells from immunized mammals in a solution containing about 30 % to about 60 % polyethylene glycol of a molecular weight between 1000 and 4000.

20. A process as claimed in claim 16, characterized in that hybridoma cell culture supernatants are screened for monoclonal antibodies binding to glycosphingolipids by glycosphingolipid blotting and positive hybridoma cells are cloned once or more by limiting dilution.

21. The use of monoclonal antibodies and/or derivatives thereof as claimed in claim 1 for the qualitative and quantitative determination and/or purification of glycoconjugates from epithelial cell membranes.

22. The use of monoclonal antibodies and/or derivatives thereof as claimed in claim 1 in the diagnosis and treatment of cancer.

23. Pharmaceutical preparations containing monoclonal antibodies and/or derivatives thereof as claimed in claim 1 and a pharmaceutical carrier.

24. The use of monoclonal antibodies and/or derivatives thereof as claimed in claim 1 for the manufacture of a pharmaceutical preparation for the treatment of mammary carcinoma.

FO 7.4/KB/we*

Claims for the Contracting State: AT

1. A process for the preparation of monoclonal antibodies and derivatives thereof, which bind to neutral glycosphingolipids produced by epithelial cells, characterized in that hybridoma cells secreting said monoclonal antibodies
a) are cultivated in vitro and the monoclonal antibodies isolated from the culture supernatant, or
b) are propagated in vivo in a suitable mammal and the monoclonal antibodies recovered from body fluids of said mammal, and, if desired,
c) the obtained monoclonal antibodies are transformed into a derivative thereof.

2. A process as claimed in claim 1, characterized in that hybridoma cells derived from Balb/c mice secreting the desired antibodies are intraperitoneally injected into Balb/c mice optionally pretreated with a hydrocarbon, after one to two weeks, ascites fluid of these mice is collected, and the monoclonal antibodies are isolated therefrom by precipitating with ammonium sulfate and/or by standard chromatographic methods.

3. A process as claimed in claim 1 or 2 for the preparation of monoclonal antibodies and derivatives thereof, which bind to neutral glycosphingolipids produced by malignant epithelial cells.

4. A process as claimed in claim 1 or 2 for the preparation of monoclonal antibodies and derivatives thereof, which bind to neutral glycosphingolipids produced by mammary carcinoma cells.

5. A process as claimed in claim 1 or 2 for the preparation of monoclonal antibodies and derivatives thereof, which bind to neutral glycosphingolipids obtained from meconium.

6. A process as claimed in claim 1 or 2 for the preparation of the monoclonal antibodies with the designation 1018S19.11, 1018S69.4, 1021S11.28 and 1022S25.17, and derivatives thereof.

7. A process as claimed in claim 1 or 2 for the preparation of the monoclonal antibodies with the designation 1018S19.11, 1018S69.4, 1021S11.28 and 1022S25.17.

8. A process as claimed in claim 1 or 2 for the preparation of the monoclonal antibody with the designation 1022S23.24, and derivatives thereof.

9. A process as claimed in claim 1 or 2 for the preparation of the monoclonal antibody with the designation 1022S23.24.

10. Hybridoma cell lines, characterized in that they secrete monoclonal antibodies which bind to neutral glycosphingolipids produced by epithelial cells.

11. Hybridoma cell lines as claimed in claim 10, characterized in that they are hybrids of mouse or rat myeloma cells and B lymphocytes of a syngeneic mouse or rat immunized with glycosphingolipids.

12. Hybridoma cell lines as claimed in claim 10 or 11, characterized in that they are hybrids of the mouse myeloma cell line Sp2/0-Ag14 and of B lymphocytes of the spleen of Balb/c mice immunized with glycosphingolipids.

13. Hybridoma cell lines as claimed in claim 10, 11 or 12, characterized in that they secrete monoclonal antibodies which bind to neutral glycosphingolipids produced by mammary carcinoma cells.

14. Hybridoma cell lines as claimed in claim 10, 11 or 12, characterized in that they secrete monoclonal antibodies which bind to neutral glycosphingolipids obtained from meconium.

15. The hybridoma cell lines with the designation 1018S19.11, 1022S25.17, 1021S11.28 and 1018S69.4, which have been deposited at the "Collection Nationale de Cultures de Microorganismes", Institut Pasteur, Paris, under the number I-331, I-332, I-333 and I-334, respectively.

16. The hybridoma cell line with the designation 1022S23.24, which has been deposited at the "Collection Nationale de Cultures de Microorganismes", Institut Pasteur, Paris, under the number I-453.

17. A process for the preparation of hybridoma cell lines as claimed in claim 10, characterized in that a suitable mammal is immunized with a glycosphingolipid mixture or antigenic derivatives thereof, antibody-producing cells of this mammal are fused with myeloma cells, the hybrid cells obtained in the fusion are cloned, and cell clones secreting the desired antibodies are selected.

18. A process as claimed in claim 17, characterized in that Balb/c mice are immunized with glycosphingolipid mixtures adsorbed on Salmonella minnesota R 595 rough mutant by three to eight parenteral injections containing from 100 µg to 500 µg glycosphingolipid mixture at intervals of one to ten days, spleen cells of the immunized mice are taken two to five days after the final booster injection, and these cells are fused with myeloma cells of a suitable cell line in the presence of a fusion promoter.

19. A process as claimed in claim 17 or 18, characterized in that antibody-producing cells derived from immunized Balb/c mice are fused with myeloma cells of the cell lines X63-Ag8.653 or Sp2/O-Ag14.

20. A process as claimed in claim 17, 18 or 19, characterized in that the myeloma cells are fused with a three- to twentyfold excess of spleen cells from immunized mammals in a solution containing about 30 % to about 60 % polyethylene glycol of a molecular weight between 1000 and 4000.

21. A process as claimed in claim 17, 18, 19 or 20, characterized in that hybridoma cell culture supernatants are screened for monoclonal antibodies binding to glycosphingolipids by glycosphingolipid blotting and positive hybridoma cells are cloned once or more by limiting dilution.

FO 7.4/KB/we*